# FASCICULE DE BREVET EUROPEEN

(11) **EP 3 077 813 B1**
(45) Date de publication et mention de la délivrance du brevet: **24.10.2018**
(21) Numéro de dépôt: 14824058.3
(22) Date de dépôt: 03.12.2014
(51) Int. Cl.: G01N 1/34, G01N 33/53, G01N 33/50

(54) **PROCÉDÉ D'ISOLEMENT D'EXOSOMES**
VERFAHREN ZUR ISOLIERUNG VON EXOSOMEN
METHOD FOR ISOLATING EXOSOMES

(30) Priorité: 03.12.2013 FR 1362021
(43) Date de publication de la demande: 12.10.2016
(73) Titulaire: Biomérieux, 69280 Marcy l'Étoile (FR); Hospices Civils De Lyon, 69002 Lyon (FR)
(72) Inventeur: OTT, Catherine, F-69800 Saint-Priest (FR); MALLET, François, F-69100 Villeurbanne (FR); GENERENAZ, Laurence, F-69800 Saint-Priest (FR)
(74) Mandataire: Agasse, Stéphane
(86) Numéro de dépôt international: PCT/FR2014/053143
(87) Numéro de publication internationale: WO 2015/082839

(56) Documents cités:
- WO-A2-2013/022995
- US-A1- 2012 309 018
- US-A1- 2013 196 355
- ANURADHA SABAPATHA ET AL: "Specific Isolation of Placenta-Derived Exosomes from the Circulation of Pregnant Women and Their Immunoregulatory Consequences", AMERICAN JOURNAL OF REPRODUCTIVE IMMUNOLOGY, vol. 56, no. 5-6, 1 novembre 2006 (2006-11-01), pages 345-355, XP055054551, ISSN: 8755-8920, DOI: 10.1111/j.1600-0897.2006.00435.x
- S. MATHIVANAN ET AL: "Proteomics Analysis of A33 Immunoaffinity-purified Exosomes Released from the Human Colon Tumor Cell Line LIM1215 Reveals a Tissue-specific Protein Signature", MOLECULAR & CELLULAR PROTEOMICS, vol. 9, no. 2, 1 February 2010 (2010-02-01), pages 197-208, XP055225655, US ISSN: 1535-9476, DOI: 10.1074/mcp.M900152-MCP200
- HINA KALRA ET AL: "Comparative proteomics evaluation of plasma exosome isolation techniques and assessment of the stability of exosomes in normal human blood plasma", PROTEOMICS, vol. 13, no. 22, 18 October 2013 (2013-10-18), pages 3354-3364, XP055293792, DE ISSN: 1615-9853, DOI: 10.1002/pmic.201300282

## Description

La présente invention concerne un procédé d'isolement d'exosomes à partir d'un échantillon biologique, et l'utilisation de ce procédé comme outil dans la caractérisation des exosomes présents dans ledit échantillon, ainsi que dans le diagnostic et le pronostic d'une pathologie et/ou du stade clinique d'une pathologie, mais aussi dans le suivi de l'évolution d'une pathologie, traitée ou non, chez un être humain ou animal.

Les exosomes sont des vésicules membranaires de 40-120 nm de diamètre, sécrétées par différents types cellulaires *in vivo.* Du fait de leur origine et leur biogénèse, les exosomes reflètent le contenu et l'état physiologique normal ou pathologique des cellules dont ils sont issus. Ils sont retrouvés dans de nombreux liquides biologiques, comme le sang, le plasma, le sérum, l'urine, la salive, le liquide céphalo-rachidien (LCR), la lymphe, la bile, les lavages broncho-alvéolaires, le sperme, le liquide synovial, le liquide amniotique, le lait maternel, les liquides d'ascites malins, ...

Leur processus de sécrétion est un processus très actif pour des cellules en prolifération telles que les cellules cancéreuses. Ils contiennent des marqueurs nucléiques et protéiques de cellules tumorales, à partir desquelles ils sont sécrétés et sont donc considérés comme des réservoirs de nouveaux biomarqueurs potentiels du cancer. En cela, leur étude présente un intérêt grandissant pour la communauté scientifique.

La limitation majeure pour leur étude réside dans l'obtention de préparations purifiées et suffisamment enrichies, à partir des différents fluides biologiques précités, avec les techniques actuelles disponibles dans l'état de l'art. La littérature souligne en effet la complexité de l'évaluation des exosomes du fait de préparations contaminées, notamment par des protéines, des fractions microsomales ou organelles co-purifiées par des techniques telles que l'ultracentrifugation ou la nanofiltration, ou de préparations trop peu concentrées en exosomes pour être analysées, issues de techniques immunologiques de séparation. A ce jour, même la combinaison de ces techniques n'a pas donné entière satisfaction.

La plupart des travaux réalisés dans le but de caractériser les exosomes sont entrepris sur des cultures de cellules malignes. Elles sont soumises à des techniques de purification des exosomes que les cellules sécrètent en cours de développement, et des profils protéomiques des exosomes isolés sont déterminés. Ainsi, selon l'article S. Mathivanan et al., Mol Cell Proteomics. 2010 Feb;9(2):197-208, les auteurs ont isolé des exosomes à partir d'une lignée cellulaire de carcinome humain du colon, LIM1215. Le milieu de culture a d'abord été soumis à une première série d'ultracentrifugations pour en séparer une population de particules d'une taille de 40-100 nm, laquelle a ensuite été engagée dans une immunopurification avec un anticorps A33 humanisé, reconnaissant spécifiquement les cellules épithéliales du colon. 394 protéines ont été identifiées, appartenant à diverses catégories de protéines, certaines étant communes à celles isolées de cultures de lignées cellulaires humaines d'urine et de culture de lignées de mastocytes murins, et révélant un rôle multifonctionnel des exosomes.

L'importance des exosomes ayant été établie, il est primordial de disposer de techniques d'isolement efficaces et spécifiques des exosomes, permettant tout à la fois d'être fiables, utilisables en routine et ne nécessitant pas de gros volumes d'échantillons biologiques.

WO2013/022995A2 divulgue un procédé d'analyse d'exosomes comprenant leur isolement à partir d'un échantillon biologique au moyen d'anticorps marqués, dirigés contre différents marqueurs de surface.

L'invention réside dans un procédé d'isolement d'exosomes qui comprend deux niveaux successifs de séparation qui reposent sur la technique de séparation par affinité. Cette séparation séquentielle confère au procédé de l'invention une haute spécificité. Elle lui permet au surplus d'être applicable à tout échantillon d'un liquide ou fluide biologique.

Le procédé de l'invention constitue un outil qui peut être utilisé en routine. Il surmonte les obstacles auxquels l'homme du métier était jusqu'à ce jour confronté, ouvrant une voie d'accès généralisée à la caractérisation des exosomes.

Le procédé de l'invention comprend au moins les deux étapes successives suivantes :
a) une première étape de purification par affinité, appliquée à un liquide biologique, utilisant au moins un anti-ligand spécifique d'un ligand générique porté par les exosomes, pour obtenir une population P d'exosomes, lesdits exosomes étant séparés dudit anti-ligand et
b) une seconde étape de purification par affinité, appliquée à la population P d'exosomes, utilisant au moins un anti-ligand spécifique d'un ligand caractéristique d'une sous-population SP d'exosomes, pour obtenir ladite sous-population SP
   d'exosomes, lesdits exosomes étant séparés dudit anti-ligand ou non.

Avant d'exposer en détail l'invention, certains termes employés dans le présent texte pour caractériser l'invention sont ci-après définis.

Les exosomes appartiennent à une fraction de nano-vésicules sécrétées par les cellules dans les liquides biologiques. Structurellement, ce sont des vésicules possédant une bi-couche lipidique comprenant en surface des protéines et des sucres. Ils sont définis par leur taille variant de 30 à 200 nm, plus particulièrement par une taille d'au moins 40 nm, voire d'au moins 50 nm, et d'au plus 150 nm, voire d'au plus 120 nm et même d'au plus 100 nm.

Les termes liquide biologique et fluide biologique sont indifféremment employés. Un liquide biologique est produit par un être humain ou animal, sain ou malade, diagnostiqué ou non. Il est recueilli ou ponctionné chez l'être humain ou animal, directement ou indirectement. Par indirectement, on comprend que l'on peut prélever chez l'être humain ou animal, des cellules ou un tissu cellulaire que l'on met en culture dans un milieu approprié dans lequel lesdites cellules excréteront des exosomes et dont tout ou partie sera prélevée pour être soumise au procédé d'isolement de l'invention. A titre d'exemples non limitatifs, on peut citer les surnageants cellulaires, les prélèvements de selles, de moelle osseuse.

Par technique de purification par affinité, on comprend une technique basée sur une interaction ou reconnaissance spécifique entre un ligand exosomal porté par l'exosome et un anti-ligand. Cette reconnaissance peut être de nature immunologique et conduire à un complexe immun, comme les interactions antigène/anticorps, épitope/anticorps, épitope/paratope, antigène/paratope..., on parle alors d'immunopurification. Cette reconnaissance peut aussi être de tout autre nature, par exemple covalente. Cette technique permet d'isoler de l'échantillon l'exosome encore lié au dit anti-ligand ou l'exosome séparé dudit anti-ligand, après élution par exemple. Une mise en oeuvre préférentielle de cette technique selon l'invention consiste à fixer, directement ou indirectement, ledit anti-ligand sur un support solide, par exemple des billes magnétiques, des membranes, des matrices de chromatographies, des microplaques, ou encore des appareils de microfluidiques.

Dans le cas d'une fixation indirecte, la fixation de l'anti-ligand au support solide peut être mise en oeuvre en utilisant une bille magnétique comme intermédiaire entre le support solide, par exemple une membrane, et l'anti-ligand. Pour ce faire, l'activation d'un aimant derrière le support solide permettra de fixer la bille magnétique au support solide, et la désactivation dudit aimant permettra de séparer du support solide les exosomes fixés à l'anti-ligand, lui-même fixé à la bille magnétique.

Par cellule normale, on comprend une cellule ne possédant pas de marqueurs détectables ou un taux détectable de marqueurs caractéristiques d'un état anormal de la cellule. Sont incluses dans cette définition, les cellules souches, notamment mésenchymateuses, neurales et hématopoïétiques, qui sécrètent des exosomes caractéristiques d'un état non différencié. Par opposition, une cellule est considérée comme anormale, quand elle possède un ou des marqueurs détectables, ou un taux détectable de marqueurs, caractéristiques d'un état différent de celui d'une cellule normale, et notamment un état pathologique de la cellule ou un état susceptible d'évoluer vers un état pathologique. Entrent notamment dans cette définition, les cellules cancéreuses, adénomateuses, infectieuses, inflammatoires, stimulées immunitairement, brûlées, et plus généralement agressées par tout type de stimulus.

Comme indiqué précédemment, malgré leur très faible taille, les exosomes présentent l'intérêt d'accumuler de très nombreuses protéines qui constituent leur traçabilité, et sont la quintessence des cellules qui les excrètent, en particulier, la nature du tissu cellulaire, l'état normal ou anormal de la cellule...

Le procédé de l'invention comporte une première étape de purification par affinité permettant d'isoler une population P d'exosomes, qui est basée sur l'existence de marqueurs spécifiques des exosomes, susceptibles d'être présents ou exposés à la surface de ceux-ci, à un stade de leur développement. Ces marqueurs qu'on appellera aussi ligands génériques, sont des antigènes, des récepteurs, des facteurs de croissance, ainsi que toute autre particule ou molécule, et toute fraction de ceux-ci, capables d'être reconnus spécifiquement par un anti-ligand.

La première étape met ainsi en oeuvre au moins un anti-ligand reconnaissant spécifiquement au moins l'un des marqueurs ou ligands ci-dessus. Selon la spécificité du ligand vis-à-vis des exosomes, et pour améliorer l'efficacité de cette étape, on peut utiliser deux anti-ligands ou plus, chacun d'eux étant spécifiques de deux ligands génériques ou plus, respectivement, ces ligands génériques étant spécifiques de la population exosomale. Selon une variante de l'invention, la première étape ne comporte qu'une seule purification, au cours de laquelle on utilise un ou plusieurs anti-ligands précités. Selon une autre variante de l'invention, la première étape peut comprendre deux sous-étapes consécutives ou plus, chacune d'elles engageant un ou plusieurs anti-ligands spécifiques d'un ou de plusieurs ligands génériques, respectivement.

Comme cela est illustré dans les exemples, la première étape conduit à l'obtention d'une population P d'exosomes qui sont séparés du ou desdits anti-ligands spécifiques d'un ou de ligands génériques des exosomes. Cette séparation est de préférence réalisée par élution, dans des conditions qui relèvent des compétences générales de l'homme du métier.

Cette première étape a) peut être appliquée à tout liquide biologique tel que défini précédemment.

Au préalable, ce liquide peut avoir été traité. Ainsi, sans sortir du cadre de l'invention, le liquide biologique peut être pré-traité par une étape de séparation physique, par taille, qui permet d'isoler une fraction du liquide biologique ne contenant pas de molécules ou de particules d'une taille supérieure à 800 nm, de préférence à 500 nm, qui sera ensuite soumise à la première étape a).

Cette étape de séparation physique peut être réalisée par toute technique appropriée telle que celles choisies parmi les techniques de centrifugation et/ou filtration, comme la filtration en série, l'ultrafiltration, la chromatographie d'exclusion par taille, et les combinaisons de ces techniques.

Cette étape de séparation physique au préalable des étapes successives de purification par affinité a pour avantage qu'elle permet d'enrichir l'échantillon avant la mise en oeuvre desdites étapes de purification par affinité, donnant des exosomes avec une purification encore plus élevée.

A l'issue de la première étape a), on obtient une population P d'exosomes qui est alors engagée dans la seconde étape b) du procédé de l'invention.

La seconde étape de purification par affinité du procédé de l'invention permettant d'isoler, depuis la population P d'exosomes, une sous-population SP d'exosomes, est basée sur l'existence de marqueurs spécifiques propres à, ou caractéristiques de certains exosomes, lesdits marqueurs étant susceptibles d'être présents ou exposés à la surface de ceux-ci, à un stade de leur développement. Ces marqueurs, qu'on appellera ligands particuliers, par opposition aux ligands génériques, sont des antigènes, des récepteurs, des facteurs de croissance, ainsi que toute autre particule ou molécule, et toute fraction de ceux-ci, capables d'être reconnus spécifiquement par un anti-ligand.

La seconde étape b) met en oeuvre au moins un anti-ligand reconnaissant spécifiquement au moins l'un des marqueurs ou ligands particuliers ci-dessus. Selon la spécificité du ligand vis-à-vis des exosomes, et pour améliorer l'efficacité de cette étape, on peut utiliser deux anti-ligands ou plus, chacun d'eux étant spécifiques de deux ligands particuliers ou plus, respectivement.

Comme cela est illustré dans les exemples, et en fonction de la technologie dans laquelle sont ensuite impliqués les exosomes isolés selon l'invention, la seconde étape conduit à l'obtention d'une sous-population SP d'exosomes qui sont liés aux dits anti-ligands spécifiques du ou desdits ligands caractéristiques d'une sous-population SP des exosomes, ou qui sont séparés desdits anti-ligands. Dans ce dernier cas, les exosomes obtenus sont libérés de tout support et sont solubles. La séparation des exosomes des anti-ligands est de préférence réalisée par élution, dans des conditions qui relèvent des compétences générales de l'homme du métier.

Selon une variante de l'invention, la seconde étape b) ne comporte qu'une seule purification, au cours de laquelle on utilise un ou plusieurs anti-ligands précités. La purification de cette seconde étape b) peut être réalisée en plusieurs sous-étapes mettant en jeu un ou plusieurs anti-ligands, concourant à l'isolement d'une même sous-population SP d'exosomes, qui sera donc de plus en plus spécifique.

Selon une autre variante de l'invention, le procédé de l'invention comprend au moins une troisième étape c) de purification par affinité, cette étape étant appliquée à ladite sous-population SP et utilisant au moins un anti-ligand spécifique d'un ligand caractéristique d'une sous-population SP' d'exosomes, la sous-population SP' étant incluse dans la sous-population SP, pour obtenir ladite sous-population SP'. A l'instar de la seconde étape b), cette troisième étape c) de purification peut comprendre des sous-étapes concourant à l'isolement d'une même sous-population SP' d'exosomes.

Bien entendu, une ou plusieurs étapes consécutives supplémentaires de purification par affinité peuvent encore venir compléter le procédé de l'invention. Dans ce cas, les exosomes seront séparés desdits anti-ligands lors des purifications par affinité précédant la dernière étape de purification par affinité, les exosomes pouvant alors être séparés ou non desdits anti-ligands lors de la dernière étape de purification.

La sous-population SP et la sous-population SP' sont des populations d'exosomes particuliers. En fonction des anti-ligands particuliers utilisés, la sous-population SP ou la sous-population SP' peut à titre d'exemple rassembler des exosomes provenant d'un même organe, d'un même tissu cellulaire, ou d'un même type de cellules. Le tissu ou les cellules d'origine peuvent être normaux ou anormaux.

En pratique et avant une illustration de la mise en oeuvre et des intérêts d'un procédé de l'invention, dans les exemples qui suivront, la population P, issue de la première étape a), peut être soumise à la seconde étape b) du procédé, en présence d'un ou de plusieurs anti-ligands spécifiques de ligands caractéristiques d'un organe pathologique, par exemple d'une tumeur de la prostate, pour isoler une sous-population SP d'exosomes caractéristiques d'une tumeur de la prostate. Dans une autre mise en oeuvre, la population P peut être soumise à la seconde étape b), en présence d'un ou de plusieurs anti-ligands spécifiques de ligands caractéristiques d'un organe, par exemple de la prostate, sans indication de l'état normal ou pathologique de l'organe. Pour affiner le procédé d'isolement, cette sous-population SP peut être soumise à une troisième étape c) avec des anti-ligands spécifiques de ligands marqueurs de cellules anormales, pour permettre l'isolement d'une sous-population SP' d'exosomes caractéristiques d'une tumeur de la prostate. On peut aussi envisager que cette sous-population SP' soit obtenue par traitement d'une population P issue de la première étape a), dans une seconde étape b) en présence d'anti-ligands spécifiques de ligands marqueurs de cellules anormales pour isoler une sous-population SP d'exosomes caractéristiques de cellules anormales ; puis, cette sous-population SP est soumise à une troisième étape c) avec des anti-ligands spécifiques de ligands caractéristiques de la prostate.

Le ou les ligands génériques et/ou le ou les ligands caractéristiques peuvent être choisis parmi des polypeptides, des protéines, des antigènes, des récepteurs, des enzymes, des facteurs de croissance, des glycolipides, des polysaccharides, et les anti-ligands spécifiques desdits ligands sont des anticorps, des fragments d'anticorps comme les fragments F(ab')2, scFv, des analogues d'anticorps, les lectines, les aptamères, des peptides. Selon l'invention, le ou au moins l'un des ligands génériques sont différents du ou d'au moins l'un des ligands caractéristiques.

Par « analogues d'anticorps », on entend des composés biologiques et/ou chimiques qui possèdent les mêmes capacités de liaison que les anticorps ou fragments d'anticorps ou des capacités de liaison similaires. En particulier, les analogues d'anticorps incluent de petites protéines qui comme les anticorps sont capables de se lier à une cible biologique permettant ainsi de la détecter, de la capturer ou tout simplement de la cibler au sein d'un organisme ou d'un échantillon biologique. Les champs d'applications de ces analogues d'anticorps sont pratiquement aussi vastes que ceux des anticorps. A titre d'exemple, on peut citer les Nanofitines™, petites protéines commercialisées par la société AFFILOGIC.

Plus particulièrement, le ligand générique est choisi parmi les protéines de la famille des tétraspanines, telles que les tétraspanines CD63, CD9, CD81 ; les protéines impliquées dans l'adhésion comme la lactadhérine (ou MFGE-8), ICAM-1 ; des protéines impliquées dans le transport et la fusion membranaire de la famille des Rab-GTPases, telles que Rab5 et Rab7 et les annexines ; et des molécules du complexe majeur d'histocompatibilité (CMH) telles que MHI, MHII.

Le ligand caractéristique d'une sous-population est de préférence choisi parmi le PSCA, l'annexine A3, le PSMA, la cavéoline, le B7H3, les protéines d'origine rétrovirale endogène comme les protéines d'enveloppe.

L'invention réside en outre dans une utilisation d'un procédé tel que décrit précédemment pour caractériser et/ou quantifier des exosomes. Comme indiqué précédemment, les exosomes isolés selon l'invention peuvent être séparés du ou des anti-ligands impliqués dans l'étape b) de purification par affinité ou dans la dernière étape de purification par affinité si d'autres étapes de purification par affinité sont effectuées.

Les exosomes isolés selon le procédé de l'invention, séparés de tout support et solubles, sont particulièrement utiles dans les applications suivantes, données à titre illustratif et non limitatif :
- Nanotechnologie/ Nanosystèmes
- Séquençage du contenu exosomal / génotypage
- Thérapie : nanovecteur et délivrance de cibles thérapeutiques
- Etudes *in vitro* : messagers cellulaires.

Le procédé de l'invention peut aussi être appliqué au diagnostic et au pronostic d'une pathologie et/ou du stade clinique d'une pathologie, mais aussi dans le suivi de l'évolution d'une pathologie, traitée ou non, chez un être humain ou animal malade ou le suivi de l'effet du traitement de cette pathologie, chez un être humain ou animal malade.

La pathologie peut être chronique ou aigüe, d'origine infectieuse ou non infectieuse. Dans une utilisation du procédé, la pathologie est un adénome, un cancer, une inflammation, une septicémie, une maladie neurologique comme les maladies d'Alzheimer, de Parkinson, la sclérose en plaques et les maladies à prions, ou une pathologie de la grossesse comme la pré-éclampsie.

Le traitement peut être un traitement médicamenteux, une radiothérapie ou une greffe.

L'invention est illustrée dans les exemples suivants où elle est appliquée à différents liquides biologiques de patients sains ou malades, à savoir atteints d'un cancer de la prostate, les anti-ligands utilisés à la seconde étape étant caractéristiques de la prostate. Ces exemples font référence aux figures suivantes :
La Figure 1 représente la détection d'exosomes en ng/µl par dosage sandwich ExoTEST Rab5/CD63 dans les sous-populations immunopurifiées par un procédé de l'invention, avec le marqueur générique CD63 et les marqueurs spécifiques PSMA (Antigène membranaire spécifique de la prostate) et cavéoline, à partir de pools de sérum de sujets sains et malades.
La Figure 2 représente la détection d'exosomes en ng/µl par dosage sandwich ExoTEST Rab5/CD63 dans des sous-populations immunopurifiées par un procédé de l'invention, avec le marqueur générique CD63 et les marqueurs AnxA3, PSCA (Antigène de cellule souche de la prostate) et B7H3 (marqueur de cellules épithéliales tumorales et du cancer de la prostate), à partir de pools de plasma CaP de sujets malades.
La Figure 3 est le résultat d'une observation par MET des exosomes sur billes de la sous-population CD63/PSMA isolée à partir de pools de sérums.
La Figure 4 est le résultat d'une observation par MET des exosomes sur billes de la sous-population CD63/AnxA3 isolée à partir de pools de sérums.
La Figure 5 est le résultat d'une observation par MET des exosomes de la sous-population CD63/AnxA3 isolée à partir de pools de sérums, après élution (ou désorption).
La Figure 6 représente la détection d'exosomes en ng/µl par dosage sandwich ExoTEST Rab5/CD63 dans des sous-populations immunopurifiées par un procédé de l'invention, avec le marqueur générique CD63 et les marqueurs PSMA, cavéoline et AnxA3 pour 2 sérums P1 et P2 de sujets atteints d'un cancer de la prostate, testés individuellement.
La Figure 7 représente la détection d'exosomes en ng/µl par dosage sandwich ExoTEST Rab5/CD63 dans des sous-populations immunopurifiées par un procédé de l'invention, avec le marqueur générique CD63 et les marqueurs CD9, PSMA, et AnxA3 pour les pools de plasma CaP et EFS.
La Figure 8 représente une comparaison entre la détection des exosomes dans une sous-population CD63/AnxA3 d'origine plasmatique (EFS et CaP) par la technique Nanosight et celle réalisée par ExoTEST.
La Figure 9 représente une comparaison entre la détection des exosomes dans une sous-population CD63/PSMA d'origine plasmatique (EFS et CaP) par la technique Nanosight et celle réalisée par ExoTEST.
La figure 10 représente la détection d'exosomes en ng/µl par dosage sandwich ExoTEST Rab5/CD63 dans des sous-populations immunopurifiées par un procédé de l'invention, avec le marqueur générique CD63 et le marqueur spécifique PSCA pour des plasmas de sujets atteints d'un cancer de la prostate (n=6) et de sujets sains (n=3).

### Exemple 1: application du procédé de l'invention à un échantillon de sang (sérum ou plasma) ou d'urine, pour isoler des exosomes caractéristiques de cellules prostatiques tumorales

### 1) Matériel

### 1.1) Echantillons de liquides biologiques

### Echantillons de sujets malades

### Pool de sérums CaP :

La méthode de purification a été réalisée au moyen d'un pool de sérums (V=2,5ml) composé de 6 échantillons de patients atteints d'un cancer de la prostate à différents stades de la maladie avec des scores de Gleason mesurant l'agressivité des cellules cancéreuses, allant de 6 à 9 (sur une échelle de 2 à 10).

### Sérums individuels CaP :

Une étude portant sur 2 sérums individuels issus du pool d'échantillons ci-dessus a été réalisée à partir d'un volume de sérum réduit à V=1,2 ml, soit une prise d'essai 2 fois moins importante. Il s'agit de 2 sérums provenant de patients (P1 et P2) ayant un score de Gleason de 7 et 8, respectivement.

### Pool de plasmas CaP :

La méthode de purification a été appliquée sur un pool de plasmas (V=2,5ml) constitué de 4 échantillons de patients atteints d'un cancer de la prostate en phase métastatique.

### Pool d'urines CaP :

La méthode de purification a été appliquée sur un pool d'urines de patients atteints d'un cancer de la prostate ayant un score de Gleason de 7, après massage post-digital rectal examination (post-DRE) (CaP) et de patients atteints d'hyperplasie bénigne de la prostate (HBP).

### Echantillons de sujets sains

### Pool de sérums EFS :

Des échantillons sériques provenant de 6 donneurs sains de l'Etablissement Français du Sang (EFS) ont permis de constituer un pool de sérum EFS (V=2,5ml) comme groupe contrôle pour l'étude.

### Pool de plasmas EFS :

Des échantillons plasmatiques provenant de 6 donneurs sains de l'EFS ont permis de constituer un pool de plasmas EFS (V=2,5ml) comme groupe contrôle pour l'étude.

### 1.2) Anticorps utilisés

Ils sont listés dans le tableau 1 ci-dessous.

**Tableau 1**

| Anticorps | Clone | Type | Cible | Marqueur |
|---|---|---|---|---|
| anti-CD63¹ | MX-49, 129,5 | Monoclonal de souris | CD63 | Générique |
| anti-CD9² | MEM-61 | Monoclonal de souris | CD9 | Générique |
| anti-PSM¹ | K1H7 | Monoclonal de souris | PSMA | Cancer prostate |
| anti-PSCA³ | 5C2 | Monoclonal de souris | PSCA | Cancer prostate |
| anti-AnxA3⁴ | 13A12G4 | Monoclonal de souris | Annexine A3 | Cancer prostate |
| anti-B7H3¹ | 4396 | Monoclonal de souris | B7H3 | Cancer agressif |
| anti-Cavéoline¹ | N-20 | Polyclonal de lapin | Cavéoline | Cancer |

| | | | | |
|---|---|---|---|---|
| 1 : fournisseur Santa Cruz Technology 2 : fournisseur Novus Biological 3 : fournisseur Sigma Aldrich 4 : produit par la société bioMérieux comme décrit dans FR2968767A1 | | | | |

### 2) Préparation des anti-ligands

Les anticorps précités sont couplés à des billes magnétiques recouvertes de streptavidine (Dynabeads® M-280 Streptavidin).

Une étape de biotinylation des anticorps est réalisée préalablement avec le kit commercial, One-step Antibody Biotinylation, commercialisé par Miltenyi Biotec, selon les recommandations du fournisseur.

Un volume de 150 µl de billes MP-280 (soit 10⁸ billes) sont prélevées puis lavées pendant 5 minutes avec 500 µl d'un tampon + 0.5% Tween. Le tube est placé sur un support aimanté afin d'éliminer le surnageant. 500 µl des anticorps monoclonaux anti-CD63 humain, anti-PSMA, anti-AnxA3, anti-cavéoline ou anti-CD9 dilués à la concentration de 20 µg/ml dans le tampon + Tween 0,05% sont ajoutés et incubés pendant 30 minutes sous agitation rotative. Pour le blocage des sites libres, une solution de biotine à 10 mM est ajoutée et incubée pendant 30 minutes sous agitation rotative. Les billes sont ensuite lavées 5 fois 5 minutes avec 500 µl de tampon + 0.5% de Tween. Le conjugué billes streptavidine MP-280/anticorps biotinylé est prêt à être mis en contact avec l'échantillon sanguin.

### 3) Pré-traitement de l'échantillon

L'échantillon sérique ou plasmatique est soumis à un traitement préalable d'ultracentrifugation consistant en deux centrifugations différentielles et une filtration comme suit.

L'échantillon (volume de 2,5 ml) est centrifugé à 500g pendant 10 minutes à +4°C afin d'éliminer les cellules sanguines et les débris cellulaires puis à 16500g pendant 20 minutes à +4°C pour soustraire microparticules et corps apoptotiques du fluide. Une étape de filtration sur 0,45 µm est réalisée pour l'élimination des vésicules extracellulaires et des agrégats protéiques de taille supérieure à 450 nm.

L'échantillon d'urine est lui aussi soumis à un traitement préalable de centrifugations différentielles, puis de filtration sur 0,45 µm. L'urine est ensuite concentrée 5X sur Vivaspin 20 (cut off 10 kD, Vivasciences).

### 4) Application de la première étape a) d'immunopurification du procédé de l'invention pour obtenir une population P

Pour cette étape, c'est un ligand générique tétraspanine qui est ciblé.

L'anti-ligand utilisé est un anticorps anti-tétraspanine, plus précisément un anti-CD63.

La première immunopurification par l'anti-tétraspanine CD63 est réalisée en 2 incubations de l'échantillon sanguin prétraité. Dans un premier temps, un batch est réalisé en incubant un volume de 1,25 ml d'échantillon avec le conjugué billes streptavidine/Ac-antiCD63 biotinylé pendant 3 heures à température ambiante sous agitation rotative. Le volume restant de 1,25 ml de sérum prétraité est incubé en batch avec le bioconjugué une nuit sous agitation rotative à température ambiante. 5 lavages de 5 minutes sont réalisés avec 500 µl de tampon + 0.5% Tween. L'étape finale d'élution est réalisée par ajout de 100 µl de tampon d'élution (0,2M glycine, HCl, pH 2,2 + 1mg/ml de BSA) après 2 minutes d'incubation avec le bioconjugué en vortexant légèrement quelques secondes. A un premier volume d'élution E1 de 100 µl sont ajoutés 14 µl de tampon de neutralisation (Tris 2M, pH 9,5). Une seconde élution + neutralisation E2 identique est réalisée. Les éluats E1 et E2 sont mélangés et un volume d'élution final de 228 µl est conservé à -80°C jusqu'à analyse.

Dans les mêmes conditions, cette étape d'immunopurification a été réalisée avec l'anticorps anti-tétraspanine, anti-CD81, sur les pools de sérums de patients malades (CaP).

Les pools d'urines CaP et HBP sont soumis à cette première étape d'immunopurification avec l'anti-tétraspanine CD63.

### 5) Application de la seconde étape b) d'immunopurification du procédé de l'invention pour obtenir une sous-population SP

Pour cette étape, les ligands particuliers sont la PSMA, la cavéoline et l'annexine A3.

Les anti-ligands utilisés sont les anticorps anti-PSMA, anti-cavéoline, anti-annexine A3.

Cette seconde étape d'immunopurification spécifique est réalisée à partir des fractions d'élution P CD63 et P CD81, respectivement, obtenues en 4). Un volume de 205 µl d'élution P est ajusté à un volume final de 1200 µl avec du tampon et fractionné en trois fois 400 µl avant d'être mis en contact avec les bioconjugués billes/Ac anti-PSMA biotinylé, billes/Ac anti-cavéoline biotinylé et billes/Ac anti-annexine A3 biotinylé pendant 3h à température ambiante sous agitation rotative. 5 lavages de 5 minutes sont réalisés avec 500 µl de tampon + 0,5% de Tween. De la même manière que pour la population P, l'élution est réalisée en deux fois avec un volume d'élution final obtenu de 228 µl conservé à -80°C jusqu'à analyse.

On obtient les sous-populations SP suivantes : CD63/PSMA, CD63/cavéoline, CD63/AnxA3, CD81/PSMA, CD81/PSCA, CD81/AnxA3.

Les pools d'urines CaP et HBP sont soumis à la seconde étape d'immunopurification avec l'anticorps anti-PSMA.

### Exemple 2 : Techniques de détection des exosomes purifiés par le procédé d'invention

### 1) Immunodétection des exosomes par ELISA, ExoTEST® (fournisseur HansaBiomed)

Afin de détecter la présence d'exosomes dans les fractions purifiées par le procédé d'invention précédemment décrit, le test ExoTEST commercialisé par la société HansaBiomed a été utilisé. Il s'agit d'un test de dosage microplaque ELISA sandwich qui utilise en phase capture un Ac monoclonal dirigé contre la protéine Rab5 (famille des Rab GTPases) et en détection un Ac monoclonal anti-CD63. Le format sandwich permet la capture spécifique des exosomes en réduisant la détection de protéines contaminantes. En outre, le test permet la quantification des exosomes à partir d'échantillons biologiques et de préparations purifiées et enrichies en exosomes, grâce à la présence d'un standard de calibration inclus dans le kit. Le dosage des échantillons a été réalisé selon les recommandations du fournisseur.

### 2) Détection des exosomes par la méthode physique, NTA (Nanoparticle Trackinq Analysis)

La société NanoSight commercialise un instrument d'analyse, le LM10-HS, permettant de mesurer et caractériser tous types de nanoparticules de taille comprise entre 10 nm et 1 µm, au sein d'un échantillon polydisperse. A l'aide d'un laser 405 nm, les nanoparticules sont excitées et leur mouvement brownien est suivi par un microscope optique et filmé par une caméra. Le logiciel fourni avec l'appareil (Nanosight 2.0) permet d'obtenir une analyse de la taille et de la concentration des différentes particules présentes dans l'échantillon.

Un seuil de détection a été déterminé à partir des mesures réalisées à partir de 6 injections de tampon PBS1X préalablement filtré 2 fois sur 0,22 µm. Ce seuil correspond à la moyenne des valeurs + 3 écart type soit 0,42.10⁸ particules/mL.

En début de chaque manipulation, la qualité du tampon PBS1X, filtré extemporanément sur 0,1 µm) utilisé pour la dilution des échantillons est contrôlée. La valeur du blanc ne doit pas dépasser le seuil de détection.

Les fractions immunopurifiées par le procédé selon l'invention sont diluées au 1/50^{e} en PBS1X pour l'analyse. Les fractions issues du pré-traitement par ultracentrifugation [cf. exemple 1, 3)] sont diluées au 1/500^{e} en PBS1X.

Pour chaque échantillon, le coefficient de variation (CV) est calculé sur les mesures de concentration et de taille (mode et mean) obtenues à la suite de 5 à 6 injections. Ces mesures permettent d'évaluer la reproductibilité de l'analyse NTA des échantillons.

### 3) Détection des exosomes par microscopie électronique à transmission (MET)

L'observation directe des exosomes en suspension après coloration négative est réalisée par microscopie électronique à transmission. On observe a) les exosomes couplés aux billes et b) les exosomes seuls, après elution.

### Exemple 3 : Application à la détection des exosomes immunopurifiés issus d'un pool de sérums selon l'invention

Les différentes techniques de détection exposées à l'Exemple 2 sont appliquées dans le présent exemple aux sous-populations d'exosomes CD63/AnxA3 et CD63/PSMA, issues du pool de sérums, de l'Exemple 1 après traitements 4) et 5) de l'Exemple 1.

### 1) Détection avec l'ExoTEST®

A partir d'un pool de sérums de patients atteints d'un cancer de la prostate CaP et d'un pool de donneurs sains (EFS), la détection des exosomes circulants isolés par immunoaffinité séquentielle générique et spécifique est réalisée au moyen du dosage ELISA sandwich ExoTEST Rab5/CD63. Une prise d'essai correspondant à ¼ du volume de chaque fraction d'isolement exosomal obtenue est dosée par ExoTEST®.

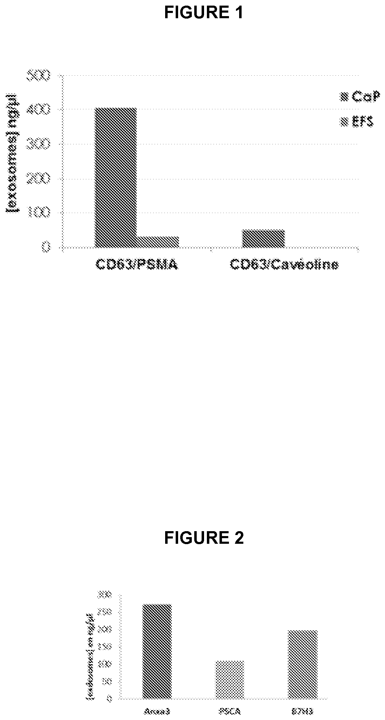

Les Figures 1 et 2 indiquent la concentration d'exosomes en ng/µl obtenue pour les différents marqueurs testés lors de la seconde immunopurification.

La figure 1 met en évidence la détection d'exosomes sériques dans les fractions suivantes :
- La population CD63/PSMA correspond à une sous-population d'exosomes issus d'un procédé de double immunopurification selon l'invention, par application d'une étape a) du procédé au pool de sérums CaP (sujets atteints d'un cancer de la prostate) et le pool de sérums EFS (sujets sains), en utilisant un anti-ligand dirigé contre le ligand générique CD63, puis application d'une étape b) à la population ainsi isolée, en utilisant un anti-ligand dirigé contre le marqueur spécifique du cancer de la prostate PSMA ;
- La population CD63/Cavéoline correspond à une sous-population d'exosomes issus d'un procédé de double immunopurification selon l'invention, par application d'une étape a) du procédé au pool de sérums CaP (sujets atteints d'un cancer de la prostate), en utilisant un anti-ligand dirigé contre le ligand générique CD63, puis application d'une étape b) à la population ainsi isolée, en utilisant un anti-ligand dirigé contre le marqueur spécifique du cancer de la prostate cavéoline.

Des concentrations supérieures en exosomes sont détectées pour le pool de sérums CaP du cancer de la prostate comparativement au pool de sérums provenant de donneurs sains.

La figure 2 met en évidence la détection d'exosomes sériques dans les fractions suivantes :
Les sous-populations CD63/AnxA3, CD63/PSCA et CD63/B7H3 d'exosomes sont obtenues à l'issue d'un procédé de double immunopurification selon l'invention, par application d'une étape a) du procédé au pool de sérums CaP (sujets atteints d'un cancer de la prostate), en utilisant un anti-ligand dirigé contre le ligand générique CD63, puis application d'une étape b) à la population ainsi isolée, en utilisant un anti-ligand dirigé contre les marqueurs spécifiques AnxA3, PSCA et B7H3, respectivement.

La spécificité du procédé d'isolement de l'invention a été vérifiée par immunodosage sandwich Luminex AnxA3 de la sous-population CD63/AnxA3. Ce dosage est très sensible, il possède une limite de détection calculée de 1,1 pg/ml. Par ce dosage, une concentration en AnxA3 de 50 pg/mL a été mesurée dans la sous-population CD63/AnxA3.

Le tableau 2 suivant donne les valeurs de concentration des exosomes détectés par ExoTEST® sur les sous-populations CD81/PSMA, CD81/PSCA, CD81/AnxA3 :

**Tableau 2**

| Sous-population | CD81/PSMA | CD81/PSCA | CD81/AnxA3 |
|---|---|---|---|
| Concentration en exosomes (ng/µl) | 763,44 | 615,33 | 690,02 |

### 2) Détection par NTA

L'analyse des sous-populations SP CD63/AnxA3 et SP CD63/PSMA issues de pool de sérums CaP, effectuée par NTA indique la concentration en exosomes ainsi que la taille du pic majoritaire et moyen dans les échantillons purifiés figurant dans le tableau 3 ci-dessous.

**Tableau 3**

| SP | Particules X10⁹/ml | CV% | Taille moyenne des pics (nm) | CV% | Taille du pic maximal (nm) | CV% |
|---|---|---|---|---|---|---|
| CD63/AnxA3 | 16,4 | 24 | 125 | 1,7 | 92 | 4,5 |
| CD63/PSMA | 24,6 | 30 | 77 | 3 | 99 | 4 |

On observe une bonne reproductibilité des profils de distribution de taille, avec pour chacun des marqueurs étudiés des tailles de pic majoritaire compris entre 90 et 100 nm et cohérentes avec la taille décrite pour les exosomes.

### 3) Détection par MET

Une caractérisation morphologique des vésicules isolées par microscopie électronique à transmission a été effectuée.

### a) Observation des exosomes fixés sur les billes :

On observe directement les vésicules capturées sur les billes (Dynabeads® M-280 streptavidine) conjuguées aux anticorps anti-Anxa3 et anti-PSMA biotinylés. Ainsi, après une première immunopurification des exosomes sériques au moyen de l'anti-CD63, l'éluat est incubé avec des billes coatées soit avec l'anti-AnxA3, soit avec l'anti-PSMA, lavées et reprises dans du PBS.

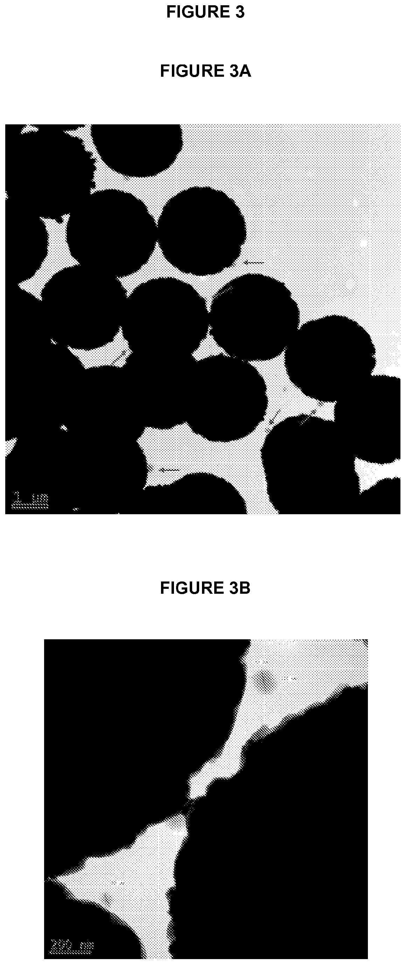

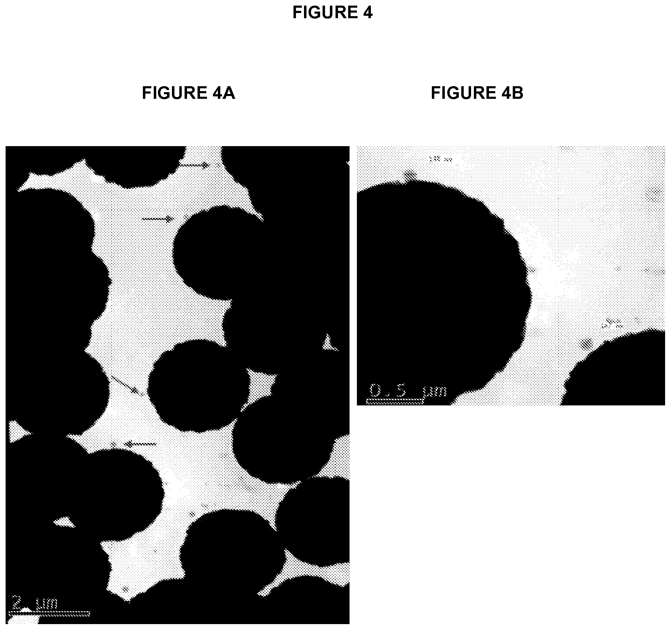

Le résultat de cette observation est illustré par les clichés ci-dessous objets de la Figure 3 (Figures 3A et 3B) pour la SP CD63/PSMA et Figure 4 (Figures 4A et 4B) pour la SP CD63/ AnxA3, sur lesquels on visualise la capture de petites vésicules à la surface des billes magnétiques.

### b) Observation des exosomes seuls après élution :

Un volume de 7,5 mL des pools de sérums a été soumis à une double immunopurification selon l'invention en utilisant les anticorps anti-CD63 pour la première étape et anti-AnxA3 et anti-PSMA, respectivement, pour la seconde étape. Chacune des sous-populations SP, CD63/AnxA3 et CD63/PSMA a été soumise à une élution par 2 x 60 µl de tampon glycine 0,2M, pH de 2,5, puis neutralisée.

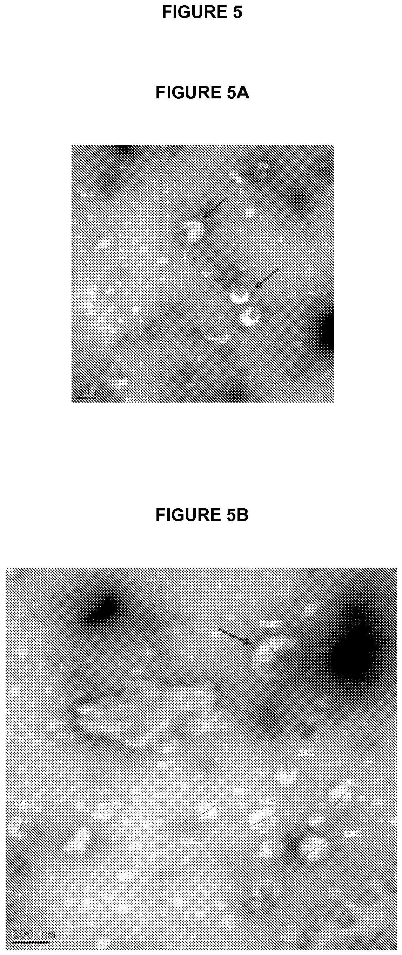

Le résultat de cette observation est illustré à la Figure 5 (Figures 5A et 5B). La morphologie en « cup-shaped » et la taille variant de 50 à 120 nm des vésicules observées sont typiques des exosomes, démontrant l'efficacité du procédé de l'invention.

### Exemple 4 : Application à la détection des exosomes immunopurifiés issus de sérums individuels selon l'invention

La technique de détection avec l'ExoTEST® exposée à l'Exemple 2 est appliquée dans le présent exemple aux sous-populations d'exosomes CD63/AnxA3 et CD63/PSMA, issues de sérums individuels CaP (sujets malades) 1.1) de l'Exemple 1 après traitements 4) et 5) de l'Exemple 1.

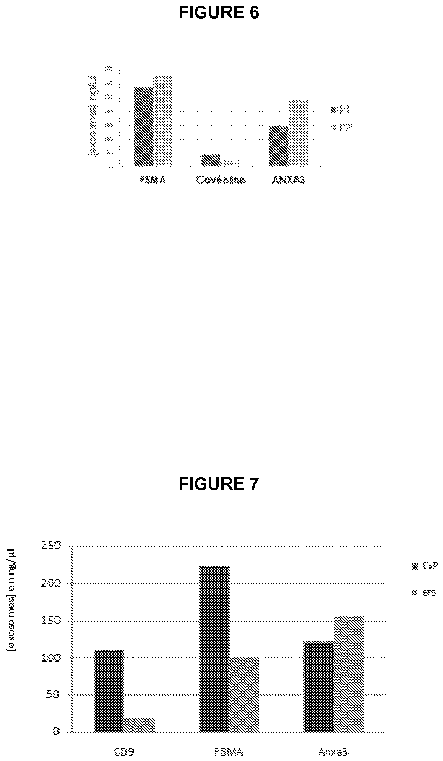

La figure 6 indique la concentration en exosomes obtenue pour les sérums individuels P1 et P2 issus du pool de sérums de patients atteints d'un cancer de la prostate. Les populations PSMA, Cavéoline et AnxA3 d'exosomes sont obtenues à l'issue d'un procédé de double immunopurification selon l'invention, par application d'une étape a) du procédé aux sérums P1 et P2, en utilisant un anti-ligand dirigé contre le ligand générique CD63, puis application d'une étape b) à la population ainsi isolée, en utilisant un anti-ligand dirigé contre les marqueurs spécifiques PSMA, Cavéoline et AnxA3, respectivement.

### Exemple 5 : Application à la détection des exosomes immunopurifiés issus d'un pool de plasmas selon l'invention

Les techniques de détection avec l'ExoTEST® et par NTA exposées à l'Exemple 2 sont appliquées dans le présent exemple aux sous-populations d'exosomes CD63/AnxA3 et CD63/PSMA, issues du pool de plasmas CaP (sujets malades) et du pool de plasmas EFS (sujets sains), de l'Exemple 1 après traitements 4) et 5) de l'Exemple 1.

### 1) Détection avec l'ExoTEST®

A partir d'un pool de plasmas de patients atteints d'un cancer de la prostate et d'un pool de donneurs sains (EFS), la détection des exosomes circulants isolés par le procédé de l'invention est réalisée au moyen du dosage ELISA sandwich ExoTEST Rab5/CD63. Les différentes fractions d'isolement exosomal obtenues sont diluées au ½ en PBS1X et dosées par ExoTEST.

La Figure 7 indique la concentration d'exosomes en ng/µl obtenue dans les fractions suivantes :
- La population CD63/PSMA correspond à une sous-population d'exosomes issus d'un procédé de double immunopurification selon l'invention, par application d'une étape a) du procédé au pool de plasmas CaP (sujets atteints d'un cancer de la prostate) et le pool de plasmas EFS (sujets sains), en utilisant un anti-ligand dirigé contre le ligand générique CD63, puis application d'une étape b) à la population ainsi isolée, en utilisant un anti-ligand dirigé contre le marqueur spécifique du cancer de la prostate PSMA ;
- La population CD63/AnxA3 correspond à une sous-population d'exosomes issus d'un procédé de double immunopurification selon l'invention, par application d'une étape a) du procédé au pool de plasmas CaP (sujets atteints d'un cancer de la prostate) et le pool de plasmas EFS (sujets sains), en utilisant un anti-ligand dirigé contre le ligand générique CD63, puis application d'une étape b) à la population ainsi isolée, en utilisant un anti-ligand dirigé contre le marqueur spécifique du cancer de la prostate Annexine A3 ;

Des concentrations supérieures en exosomes sont observées pour le pool de plasmas CaP comparativement au pool de plasmas des donneurs sains pour le marqueur PSMA.

De manière intéressante, on observe une différence de concentration en exosomes entre le pool de plasmas de donneurs sains et celle du pool de plasmas CaP pour le marqueur AnxA3, la première étant supérieure à la seconde.

### 2) Détection par NTA

L'analyse des SP CD63/AnxA3 et CD63/PSMA issues de pool de plasmas CaP et de pool de plasmas EFS, effectuée par NTA indique la concentration en exosomes ainsi que la taille du pic majoritaire et moyen dans les échantillons purifiés figurant dans le tableau 4 ci-dessous.

**Tableau 4**

| SP | Particules X10⁹/ml | CV% | Taille moyenne des pics (nm) | CV% | Taille du pic maximal (nm) | CV% |
|---|---|---|---|---|---|---|
| CD63/AnxA3 EFS | 3,1 | 10,8 | 140 | 32,4 | 108 | 14,1 |
| CD63/AnxA3 CaP | 2,7 | 12,8 | 124 | 2,8 | 85 | 7,3 |
| CD63/PSMA EFS | 8,5 | 34,8 | 163 | 19,9 | 122 | 15,5 |
| CD63/PSMA CaP | 14,2 | 15,9 | 176 | 7,5 | 133 | 14,4 |

La taille des pics majoritaires est cohérente avec la taille décrite des exosomes.

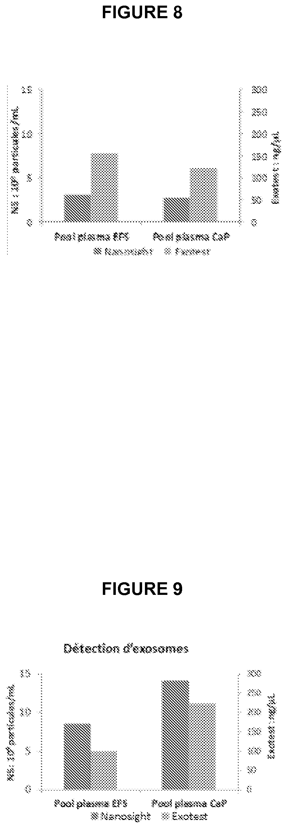

Les figures 8 et 9 illustrent une comparaison entre les deux techniques ExoTEST et NTA, pour respectivement les sous-populations SP CD63/AnxA3 et SP CD63/PSMA. On observe une adéquation entre ces deux techniques de détection.

### Exemple 6 : Application à la détection des exosomes immunopurifiés issus de plasmas selon l'invention

La technique de détection avec l'ExoTEST® exposée à l'Exemple 2 est appliquée dans le présent exemple aux sous-populations d'exosomes CD63/PSCA, issues de plasmas de patients atteints d'un cancer de la prostate et de sujets sains, respectivement.

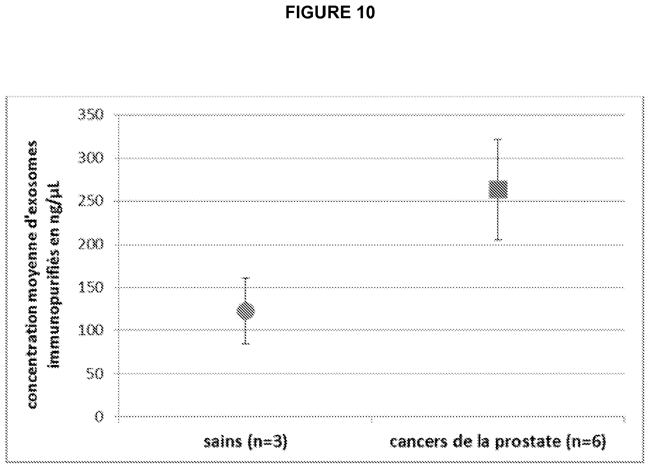

La figure 10 indique la concentration moyenne en exosomes des sous-populations SP CD63/PSCA isolées pour les groupes de patients sains (n=3) et pour les groupes de patients atteints d'un cancer de la prostate (n=6), respectivement. On observe un niveau d'exosomes plasmatique plus élevé chez les patients malades que chez les sujets sains.

### Exemple 7 : Application à la détection des exosomes immunopurifiés issus d'un pool d'urines selon l'invention

Les fractions immunopurifiées ci-dessus sont dosées par dosage ELISA ExoTEST. Les résultats sont présentés dans le tableau 5 ci-dessous.

**Tableau 5**

| | **CD63/PSMA** |
|---|---|
| IP2 | **ELISA ExoTest [exosomes] ng/ml** |
| **Pool Urine HBP** | 513 |
| **Pool Urine CaP** | 1366 |

Une concentration deux fois supérieure en exosomes est détectée pour le pool CaP versus le pool HBP. Des exosomes urinaires présentant le marqueur prostatique de surface PSMA sont donc deux fois plus nombreux dans l'urine de patients atteints d'un CaP.

### Exemple 8 : Dosage spécifique du marqueur antigène spécifique de prostate total (tPSA)

Le tPSA a été dosé dans les pools de sérums CaP et EFS ainsi que dans les fractions exosomales purifiées afin de vérifier la qualité des fractions exosomales obtenues après double immunopurification.

Pour cela, une adaptation du dosage sandwich TPSA VIDAS utilisant en capture l'anticorps monoclonal 12C11C3 et en détection l'anticorps 11E5C6 biotinylé a été faite en format Luminex. La technique permet à partir de microsphères en suspension de détecter et de quantifier plusieurs biomolécules dans un même échantillon de faible volume avec une sensibilité élevée.

Des billes magnétiques de 5,6 µm de diamètre, présentant une adresse spectrale basée sur leur contenu rouge/infrarouge ont servi de support pour le dosage. Une quantité de 9 µg d'anticorps de capture 12C11C3 a été greffé à la surface des billes magnétiques (Bio-Rad, Bio-Plex Pro Magnetic COOH Beads Amine Coupling Kit) selon les instructions du fournisseur.

Pour le dosage des pools sériques et plasmatiques, les échantillons sont dilués au 1/5^{e} en tampon TBST. Pour les fractions exosomales purifiées, le dosage de tPSA est réalisé avec 1/8 du volume de la fraction exosomale éluée.

Les échantillons sont incubés en plaque 96 puits (Bio-Rad, 171025001) en présence de 5000 billes couplées à l'anticorps de capture pendant 2 heures à 37°C, 650 tours/minute, et à l'abri de la lumière. Entre chaque étape, les puits sont lavés 3 fois en TBST 0,05%. La détection est réalisée avec 100 µl d'anticorps secondaire 11E5C6 biotinylé à la concentration de 0,005 µg/ml pendant 1 heure à 37°C sous agitation. La révélation du complexe immun a lieu par incubation de 100 µl de solution de streptavidine couplée à la phycoérythrine (RPE) à la concentration de 2 µg/ml (Dako) pendant 30 minutes à 37°C sous agitation. L'étape finale consiste à resuspendre les complexes immuns dans 100 µl de TBS pour une analyse de fluorimétrie en flux effectuée par l'automate Bio-Plex 200 (Bio-Rad). Chaque bille va subir une double excitation par un laser rouge (633 nm) pour son identification et un laser vert (532 nm) pour la quantification de l'analyte par mesure du conjugué fluorescent.

La limite de détection analytique du dosage tPSA développé sur Luminex atteint une excellente sensibilité de 1,1 pg/ml de tPSA.

Les résultats du dosage dans le sérum CaP sont présentés dans le tableau 6 ci-dessous.

**Tableau 6**

| | [tPSA] en ng/ml | | |
|---|---|---|---|
| | Pool sérums | Fraction purifiée IP2 | |
| | | PSMA | AnxA3 |
| CaP | 2,98 | 0 | 0 |

Les résultats du dosage Luminex tPSA montrent que le marqueur tPSA sérique est détecté à la concentration de 2,98 ng/ml dans le pool de sérum CaP. Après purification du pool sérique, le marqueur tPSA n'est pas ou très peu détecté dans les fractions exosomales purifiées. Ce résultat indique la qualité de la fraction exosomale purifiée obtenue par le procédé d'invention, avec l'élimination du marqueur protéique soluble tPSA des préparations purifiées.

### Exemple 9 : Répétabilité et reproductibilité du procédé

La répétabilité, c'est-à-dire la variabilité intra-série, et la reproductibilité, c'est-à-dire la variabilité inter-séries et inter-jours, du procédé d'isolement de l'invention ont été étudiées sur 4 jours.

Le test est réalisé pour l'isolement de sous-populations CD63/AnxA3 et CD63/PSMA d'exosomes à partir d'un pool de sérum de patients malades. L'immunodétection des exosomes est effectuée par ExoTEST et les résultats figurent dans le tableau 7 ci-dessous.

**Tableau 7**

| Ligand | Statistique | Répétabilité | Reproductibilité |
|---|---|---|---|
| AnxA3 | Ecart-type | 17,95 | 27,78 |
| | CV (%) | 4,87 | 7,54 |
| PSMA | Ecart-type | 129,89 | 94,80 |
| | CV (%) | 24,63 | 18,62 |

On observe pour le ligand AnxA3 une très bonne répétabilité, avec un CV de 4,87 %. Pour ce marqueur, la reproductibilité inter-jour indique également un très bon CV de 7,5%.

Des variations un peu plus importantes mais parfaitement acceptables sont observées pour le marqueur spécifique PSMA.

## Revendications

1. Procédé d'isolement d'exosomes à partir d'un liquide biologique, **caractérisé en ce qu'**il comprend au moins deux étapes successives de purification par affinité suivantes :
a) une première étape utilisant au moins un anti-ligand spécifique d'un ligand générique porté par les exosomes, pour obtenir une population P d'exosomes, lesdits exosomes étant séparés dudit anti-ligand et ledit ligand étant porté par ladite population P d'exosomes, et
b) une seconde étape, appliquée à la population P d'exosomes, utilisant au moins un anti-ligand spécifique d'un ligand caractéristique d'une sous-population SP d'exosomes, pour obtenir ladite sous-population SP d'exosomes, lesdits exosomes étant séparés dudit anti-ligand ou non.

2. Procédé selon la revendication 1, **caractérisé en ce que** la seconde étape utilise au moins deux anti-ligands, chacun d'eux étant spécifique d'un ligand respectivement caractéristique d'une sous-population SP1 et SP2.

3. Procédé selon la revendication 1 ou 2, **caractérisé en ce que** la première étape utilise au moins deux anti-ligands, chacun d'eux étant spécifique d'un ligand respectivement générique des exosomes.

4. Procédé selon l'une quelconque des revendications 1 à 3, **caractérisé en ce qu'**il comprend au moins une troisième étape c) de purification par affinité, cette étape étant appliquée à ladite sous-population SP et utilisant au moins un anti-ligand spécifique d'un ligand caractéristique d'une sous-population SP' d'exosomes, la sous-population SP' étant incluse dans la sous-population SP, pour obtenir ladite sous-population SP'.

5. Procédé selon la revendication 4, **caractérisé en ce que** la troisième étape utilise au moins deux anti-ligands, chacun d'eux étant spécifique d'un ligand respectivement caractéristique d'une sous-population SP'1 et SP'2.

6. Procédé selon l'une quelconque des revendications 1 à 5, **caractérisé en ce que** le ligand générique des exosomes et/ou le ou les ligands caractéristiques d'une sous-population SP ou SP' d'exosomes sont choisis parmi les ligands présents à la surface des exosomes.

7. Procédé selon l'une quelconque des revendications 1 à 6, caractérisé en la ou les sous-populations d'exosomes sont des sous-populations d'exosomes provenant d'un même organe.

8. Procédé selon l'une quelconque des revendications 1 à 7, **caractérisé en ce que** la ou les sous-populations d'exosomes sont des sous-populations d'exosomes provenant d'un même tissu.

9. Procédé selon l'une quelconque des revendications 1 à 8, **caractérisé en ce que** la ou les sous-populations d'exosomes sont des sous-populations d'exosomes provenant d'un même type de cellules.

10. Procédé selon l'une quelconque des revendications 1 à 9, **caractérisé en ce que** la ou les sous-populations d'exosomes sont des sous-populations d'exosomes provenant d'un tissu ou de cellules sains ou d'un tissu ou de cellules anormaux.

11. Procédé selon la revendication 10, **caractérisé en ce que** le tissu ou les cellules anormaux sont tumoraux.

12. Procédé selon l'une quelconque des revendications 1 à 11, **caractérisé en ce que** le ligand est caractéristique d'une sous-population SP d'exosomes provenant de la prostate.

13. Procédé selon l'une quelconque des revendications 1 à 12, **caractérisé en ce que**, préalablement à la première étape a), le liquide biologique est traité par une étape de séparation physique, par taille, qui permet d'isoler une fraction du liquide biologique ne contenant pas de molécules ou de particules d'une taille supérieure à 800 nm, de préférence à 500 nm, qui sera soumise à la première étape a).

14. Procédé selon l'une quelconque des revendications 1 à 13, **caractérisé en ce que**, à l'issue de l'étape a), les exosomes sont séparés de l'anti-ligand par élution.

15. Procédé selon l'une quelconque des revendications 1 à 14, **caractérisé en ce que**, à l'issue de l'étape b), les exosomes sont séparés de l'anti-ligand par élution.

16. Utilisation d'un procédé selon l'une quelconque des revendications 1 à 15, pour caractériser et/ou quantifier des exosomes.

17. Utilisation selon la revendication 16, pour le diagnostic et le pronostic d'une pathologie et/ou du stade clinique d'une pathologie, et pour le suivi de l'évolution d'une pathologie, traitée ou non, ou pour le suivi de l'efficacité du traitement d'une pathologie, chez un être humain ou animal.

18. Utilisation selon la revendication 17, **caractérisée en ce que** la pathologie est chronique ou aigüe, d'origine infectieuse ou non infectieuse.

19. Utilisation selon la revendication 17 ou 18, **caractérisée en ce que** le traitement est un traitement médicamenteux, une radiothérapie ou une greffe.

## Patentansprüche

1. Verfahren zur Isolierung von Exosomen, ausgehend von einer biologischen Flüssigkeit, **dadurch gekennzeichnet, dass** es mindestens zwei folgende sukzessive Schritte des Affinitätsreinigens umfasst:
a) einen ersten Schritt, der mindestens einen Antiliganden verwendet, der spezifisch für einen generischen Liganden, getragen von den Exosomen ist, um eine Population P von Exosomen zu erhalten, wobei die Exosomen vom Antiliganden getrennt sind und der Ligand von der Population P von Exosomen getragen wird, und
b) einen zweiten Schritt, angewendet auf die Population P von Exosomen, der mindestens einen Antiliganden verwendet, der spezifisch für einen Liganden ist, der charakteristisch für eine Unterpopulation SP von Exosomen ist, um die Unterpopulation SP von Exosomen zu erhalten, wobei die Exosomen von dem Antiliganden getrennt sind oder nicht.

2. Verfahren nach Anspruch 1, **dadurch gekennzeichnet, dass** der zweite Schritt mindestens zwei Antiliganden verwendet, wobei jeder von ihnen spezifisch für einen Liganden bzw. charakteristisch für eine Unterpopulation SP1 und SP2 ist.

3. Verfahren nach Anspruch 1 oder 2, **dadurch gekennzeichnet, dass** der erste Schritt mindestens zwei Antiliganden verwendet, die jeweils spezifisch für einen Liganden bzw. generisch für die Exosomen sind.

4. Verfahren nach einem der Ansprüche 1 bis 3, **dadurch gekennzeichnet, dass** es mindestens einen dritten Schritt c) des Affinitätsreinigens umfasst, wobei dieser Schritt auf die Unterpopulation SP angewendet wird und mindestens einen Antiliganden verwendet, der charakteristisch für eine Unterpopulation SP' von Exosomen ist, wobei die Unterpopulation SP' in der Unterpopulation SP eingeschlossen ist, um die Unterpopulation SP' zu erhalten.

5. Verfahren nach Anspruch 4, **dadurch gekennzeichnet, dass** der dritte Schritt mindestens zwei Antiliganden verwendet, wobei jeder von ihnen spezifisch für einen Liganden bzw. charakteristisch für einen Unterpopulation SP'1 und SP'2 ist.

6. Verfahren nach einem der Ansprüche 1 bis 5, **dadurch gekennzeichnet, dass** der Ligand, der generisch für die Exosomen ist, und/oder der oder die Ligand(en), die charakteristisch für eine Unterpopulation SP oder SP' von Exosomen sind, ausgewählt sind aus den Liganden, die auf der Oberfläche der Exosomen vorhanden sind.

7. Verfahren nach einem der Ansprüche 1 bis 6, **dadurch gekennzeichnet, dass** die Unterpopulation(en) von Exosomen Unterpopulationen von Exosomen sind, die aus einem gleichen Organ stammen.

8. Verfahren nach einem der Ansprüche 1 bis 7, **dadurch gekennzeichnet, dass** die Unterpopulation(en) von Exosomen Unterpopulationen von Exosomen sind, die aus einem gleichen Gewebe stammen.

9. Verfahren nach einem der Ansprüche 1 bis 8, **dadurch gekennzeichnet, dass** die Unterpopulation(en) von Exosomen Unterpopulationen von Exosomen sind, die vom gleichen Typ von Zellen stammen.

10. Verfahren nach einem der Ansprüche 1 bis 9, **dadurch gekennzeichnet, dass** die Unterpopulation(en) von Exosomen Unterpopulationen von Exosomen sind, die aus einem Gewebe oder aus gesunden Zellen oder aus einem Gewebe oder anormalen Zellen stammen.

11. Verfahren nach Anspruch 10, **dadurch gekennzeichnet, dass** das Gewebe oder die anormalen Zellen Tumore sind.

12. Verfahren nach einem der Ansprüche 1 bis 11, **dadurch gekennzeichnet, dass** der Ligand charakteristisch für eine Unterpopulation SP von Exosomen ist, die aus der Prostata stammen.

13. Verfahren nach einem der Ansprüche 1 bis 12, **dadurch gekennzeichnet, dass** vor dem ersten Schritt a) die biologische Flüssigkeit durch einen Schritt des physischen Trennens nach Größe behandelt wird, der ermöglicht, eine Fraktion der biologischen Flüssigkeit zu isolieren, die keine Moleküle oder Partikel von einer Größe über 800 nm, vorzugsweise 500 nm, enthält, die dem ersten Schritt a) unterzogen wird.

14. Verfahren nach einem der Ansprüche 1 bis 13, **dadurch gekennzeichnet, dass** am Ausgang von Schritt a) die Exosome vom Antiliganden durch Elution getrennt werden.

15. Verfahren nach einem der Ansprüche 1 bis 14, **dadurch gekennzeichnet, dass** am Ausgang von Schritt b) die Exosome vom Antiliganden durch Elution getrennt werden.

16. Verwendung eines Verfahrens nach einem der Ansprüche 1 bis 15, um Exosome zu kennzeichnen und/oder Exosome zu quantifizieren.

17. Verwendung nach Anspruch 16 zur Diagnose und zur Prognose einer Pathologie und/oder des klinischen Zustands einer Pathologie und zur Nachverfolgung einer Pathologie, behandelt oder nicht, oder zur Nachverfolgung der Wirksamkeit der Behandlung einer Pathologie bei einem Menschen oder Tier.

18. Verwendung nach Anspruch 17, **dadurch gekennzeichnet, dass** die Pathologie chronisch oder akut, infektiösen oder nicht infektiösen Ursprungs ist.

19. Verwendung nach Anspruch 17 oder 18, **dadurch gekennzeichnet, dass** die Behandlung eine medikamentöse Behandlung, eine Radiotherapie oder eine Transplantation ist.

## Claims

1. A method for isolating exosomes from a biological liquid, **characterized in that** it comprises at least the following two successive steps of affinity purification:
a) a first step using at least one specific anti-ligand of a generic ligand carried by the exosomes, so as to obtain a population P of exosomes, said exosomes being separated from said anti-ligand, and said ligand being carried by said population P of exosomes, and
b) a second step, applied on the population P of exosomes, using at least one specific anti-ligand of a characteristic ligand of a subpopulation SP of exosomes, so as to obtain said subpopulation SP of exosomes, said exosomes being separated or not from said anti-ligand.

2. The method according to claim 1, **characterized in that** the second step uses at least two anti-ligands, each of which being specific to a ligand respectively characteristic of a subpopulation SP1 and SP2.

3. The method according to claim 1 or 2, **characterized in that** the first step uses at least two anti-ligands, each of which being specific to a ligand respectively generic of the exosomes.

4. The method according to any one of claims 1 to 3, **characterized in that** it comprises at least one third step c) of affinity purification, this step being applied on said subpopulation SP and using at least one specific anti-ligand of a ligand characteristic of a sub-population SP' of exosomes, the subpopulation SP' being included in the subpopulation SP, so as to obtain said subpopulation SP'.

5. The method according to claim 4, **characterized in that** the third step uses at least two anti-ligands, each of which being specific to a ligand respectively characteristic of a subpopulation SP'1 and SP'2.

6. The method according to any one of claims 1 to 5, **characterized in that** the generic ligand of the exosomes and/or the ligand(s) characteristic of a subpopulation SP or SP' of exosomes are selected among the ligands present at the surface of the exosomes.

7. The method according to any one of claims 1 to 6, **characterized in that** the subpopulation(s) of exosomes is/are subpopulations of exosomes coming from the same organ.

8. The method according to any one of claims 1 to 7, **characterized in that** the subpopulation(s) of exosomes is/are subpopulations of exosomes coming from the same tissue.

9. The method according to any one of claims 1 to 8, **characterized in that** the subpopulation(s) of exosomes is/are subpopulations of exosomes coming from the same type of cells.

10. The method according to any one of claims 1 to 9, **characterized in that** the subpopulation(s) of exosomes is/are subpopulations of exosomes coming from healthy tissue or cells or from abnormal tissue or cells.

11. The method according to claim 10, **characterized in that** the abnormal tissue or cells are tumoral.

12. The method according to any one of claims 1 to 11, **characterized in that** the ligand is characteristic of a subpopulation SP of exosomes coming from the prostate.

13. The method according to any one of claims 1 to 12, **characterized in that**, prior to the first step a), the biological liquid is treated through a step of physical separation, by size, which allows isolating a fraction of the biological liquid which does not contain molecules or particles of a size larger than 800 nm, preferably 500 nm, which will be subjected to the first step a).

14. The method according to any one of claims 1 to 13, **characterized in that**, upon completion of step a), the exosomes are separated from the anti-ligand by elution.

15. The method according to any one of claims 1 to 14, **characterized in that**, upon completion of step b), the exosomes are separated from the anti-ligand by elution.

16. A use of a method according to any one of claims 1 to 15, for characterizing and/or quantifying exosomes.

17. The use according to claim 16, for the diagnosis and prognosis of a pathology and/or of the clinical stage of a pathology, and for monitoring the evolution of a pathology, whether treated or not, or for monitoring the effectiveness of the treatment of a pathology, in a human or an animal.

18. The use according to claim 17, **characterized in that** the pathology is chronic or acute, of infectious or non-infectious origin.

19. The use according to claim 17 or 18, **characterized in that** the treatment is a medicinal treatment, a radiotherapy or a graft.
